# EUROPEAN PATENT SPECIFICATION

(11) **EP 1 545 657 B1**
(45) Date of publication and mention of the grant of the patent: **02.11.2006**
(21) Application number: 03793604.4
(22) Date of filing: 08.09.2003
(51) Int. Cl.: A61M 5/142, A61M 5/14, B01J 19/00, B01L 3/00, B81B 1/00

(54) **FLOW RESTRICTOR WITH SAFETY FEATURE**
FLUSSBESCHRÄNKER MIT SICHERHEITSMERKMAL
RESTRICTEUR DE DEBIT A DISPOSITIF DE SECURITE

(30) Priority: 09.09.2002 EP 02388059; 09.09.2002 EP 02388060; 09.09.2002 EP 02388061
(43) Date of publication of application: 29.06.2005
(73) Proprietor: NOVO NORDISK A/S, 2800 Bagsvaerd (DK)
(72) Inventor: PEDERSEN, Per, Elg rd, DK-4690 Haslev (DK); BENDSEN, Henrik, DK-1656 Copenhagen (DK)
(86) International application number: PCT/DK2003/000574
(87) International publication number: WO 2004/022136

(56) References cited:
- EP-A- 0 110 004
- EP-A- 0 344 895
- EP-A- 0 410 289
- EP-A- 0 951 916
- WO-A-01/25138
- WO-A-01/56633
- DE-A- 19 548 537
- DE-A- 19 623 779
- DE-C- 19 825 912
- DE-U- 29 701 416
- GB-A- 2 049 225
- US-A- 2 605 765
- US-A- 4 299 220
- US-A- 4 588 319
- US-A- 4 626 244
- US-A- 4 662 914
- US-A- 4 743 235
- US-A- 5 500 071
- US-A- 5 603 351
- US-A- 5 935 430

## Description

### FIELD OF THE INVENTION

The present invention relates to means providing improved functionality and reliability in respect of the transfer of fluids and in particular aspects to flow restrictors suitable for the controlled transfer of fluid from a first compartment to a second compartment. In specific embodiments, the invention relates to safety arrangements reducing the risks associated with diminished flow resistance through the flow restrictor. Such flow restrictors are suitable for the application in fluid delivery devices of the bleeding hole type, such devices being suitable in particular for the in situ administration of a therapeutic drug preparation over a prolonged period of time, however, such devices may also be used in areas such as biochemistry, microbiology and chemical analysis.

### BACKGROUND OF THE INVENTION

In the disclosure of the present invention reference is mostly made to the treatment of diabetes by injection or infusion of insulin, however, this is only a preferred use of the present invention.

Diabetes mellitus is the common name for at least 2 different diseases, one characterised by immune system mediated specific pancreatic beta cell destruction (insulin dependent diabetes mellitus (IDDM) or type 1 diabetes), and another characterised by decreased insulin sensitivity (insulin resistance) and/or a functional defect in beta cell function (non-insulin dependent diabetes mellitus (NIDDM) or type 2 diabetes).

The principal treatment of type 1 diabetes is straight forward substitution of the missing insulin secretion, whereas treatment of type 2 is more complicated. More specifically, in early stages of type 2 diabetes treatment a number of different types of drugs can be used, e.g. drugs which increase insulin sensitivity (ciglitazones), decrease hepatic glucose output (e.g. metformin), or reduce glucose uptake from the gut (alfa glucosidase inhibitors), as well as drugs which stimulate beta cell activity (e.g. sulfonylurea/meglitinides). However, the above-described deterioration is reflected in the fact that beta cell stimulators will eventually fail to stimulate the cell, and the patient has to be treated with insulin, either as mono therapy, or in combination with oral medication in order to improve glucose control.

Currently, there are two principal modes of daily insulin therapy, the first mode including syringes and insulin injection pens. These devices are simple to use and are relatively low in cost, but they require a needle stick at each injection, typically 3-4 times or more per day. The second mode is infusion pump therapy, which entails the purchase of a relatively expensive pump, for which reason the initial cost of the pump is a barrier to this type of therapy. Although more complex than syringes and pens, the pump offer the advantages of continuous infusion of insulin, precision in dosing and optionally programmable delivery profiles and user actuated bolus infusions in connections with meals.

Basically the infusion pump comprises means for allowing the contained insulin to be transferred to the body of the patient. These means may take any desirable form providing the desired function, but presently pump arrangements comprising a conveying arrangement connected to the reservoir (i.e. an outlet to be associated with needle infusion means) and including a pressure or suction generating device for feeding the liquid contained in the reservoir by pressure or suction application from the reservoir to the body are preferred for transferring the insulin contained in the reservoir to the patient. In this respect a number of different principles may be utilized, e.g. osmotic pumps as known from for example US patents 4,340,048 and 4,552,561, piston pumps as known from for example US patent 5,858,001, membrane pumps as known from for example US patent 6,280,148, flow restrictor pumps (also known as bleeding hole pumps) as known from for example US patents 2,605,765 and 5,957,895, and gas generating pumps as known from for example US patent 5,527,288, which all in the last decades have been proposed for use in durable (refillable) and/or disposable (prefilled) drug infusion systems. As some of these principles may not be considered to be pumps in the traditional sense, it may be more appropriate to generally describe these devices as delivery means for fluid, however, in the following description the traditional term pump will be used. DE 198 25 912 discloses an implantable infusion pump in which a number of channels are formed between two members. US patent 4,662,914 discloses an interface using a capillary for connecting a gas chromatograph and a mass spectrometer.
Of the above pump principles, the present invention is especially useful for the bleeding hole type. Basically, this principle provides a means for establishing a flow of a fluid at a desired rate by applying a force to a liquid to thereby force the liquid through a flow restrictor, the flow rate being determined by the pressure generated on the fluid by the applied force, the flow resistance in the flow restrictor *per se* and the viscosity of the fluid. As the flow resistance is determined in combination by the structure of the flow restrictor (i.e. its configuration and dimensions) and the viscosity of the fluid, the term "flow resistance in the flow restrictor *per se*" refers to the former component. When in the following reference is made to the "flow resistance in the flow restrictor" this is to be understood as the "flow resistance in the flow restrictor *per se".* For the purpose of expelling a drug from a reservoir, two variants of this principle have been described.

In a first variant the drug to be infused is contained in a reservoir in fluid communication with an outlet through a flow restrictor. When the drug is pressurized by an actuating (driving) force it is forced through the flow restrictor at a rate determined by the applied force, the flow resistance in the flow restrictor and the viscosity of the drug, see for example US patent 5,957,895 which discloses an infusion device in which the driving force is provided by a drug reservoir formed between two Belleville springs, the flow restrictor being provided by the through-going channel of a capillary tube, or WO 02/15965 disclosing an infusion device in which the flow restrictor is in the form of a tortuous serpentine-formed channel established between two members. In the latter the flow resistance is selectable just as a bolus function is provided. Also US patent 5,993,414 discloses an infusion device utilizing a tortuous path flow restrictor.

In a second variant an infusion device comprises a first cavity containing a drive fluid, a flow restrictor comprising a flow channel, a second cavity in fluid communication with the first cavity through the flow channel, and a drug reservoir containing the drug to be infused, where the second cavity and the drug reservoir is arranged such that the volume of the drug reservoir diminishes when the volume of the second cavity increases. Further, drive means for expelling the drive fluid from the first to the second cavity through the flow restrictor is provided, whereby drug is expelled from the drug reservoir. The force-transmitting interface between the second cavity and the drug reservoir could be described as a secondary pump actuated by the drive means. As appears, the drug flow rate will be determined by the pressure generated by the applied force, the flow resistance in the flow restrictor and the viscosity of the drive fluid. Advantages of the second variant are that the (delicate) drug does not have to be forced through the narrow flow restrictor and that a drive fluid having a high viscosity can be used thereby allowing a flow restrictor with a smaller flow resistance to be used which will normally be less expensive to manufacture. Examples of the second variant are disclosed in US patent 2,605,765 and German published patent application 25 52 446.

In the above referred infusion devices using the bleeding hole principle, it has been an object to provide a constant infusion rate which has been achieved using force generating means providing a near-constant force, e.g. different forms of springs, this in combination with liquid reservoirs having near-constant resistance characteristics during expulsion of the contained liquid. However, it is also possible to use the bleeding hole principle in combination with flow rate controlling means as known from the infusion device described in EP 1 177 802, this infusion device comprising processor controlled valve means which opens and closes the drug flow generated using a bleeding hole pump.

Although the bleeding hole principle thus has been proposed for a variety of drug infusion devices during the last five decades, it appears that the risks associated with this principle have until now not been properly identified. More specifically, the present inventors have realized that a pump based on the bleeding hole principle has a potential run-away risk if the flow restrictor is by-passed by alternative flow paths having a lower flow resistance, e.g. due to manufacturing errors or damage, which would result in the pump rate being too high which ultimately may result in drug overdosing.

A solution to this problem could be the provision of a flow sensor arranged close to the drug outlet and cooperating with alarm means, however, the provision of such additional systems would add considerably to the manufacturing costs, and be a major obstacle to the implementation of this pump principle for a prefilled, disposable infusion device.

### DISCLOSURE OF THE INVENTION

Having regard to the above-identified problems, it is an object of the present invention to provide a flow restrictor suitable for use in a bleeding hole pump, which provides improved safety and can be manufactured in a cost-effective manner.

Flow restrictors may be provided by many different structures, however, flow restrictors in which (i) a flow channel is established between two cooperating portions of at least two members, and (ii) a flow channel is provided in the form of a capillary tube, appear especially suitable for use in medical delivery devices due to there simple construction and well defined properties. Indeed, in accordance with its function a flow channel is closed along its length forming an enclosed passage. Correspondingly, a flow channel may alternatively be termed a flow conduit, however, in the present application primarily the term flow channel is used.

When implementing the first principle, a given flow resistance may in principle be established by a narrow tubular channel of any desired length, however, a very short channel would have to be correspondingly narrow which would be difficult and expensive to manufacture. Therefore, a longer channel with a larger cross-sectional area is normally preferred, however, as this results in a relatively long channel it is necessary to "pack" the channel in an appropriate way, typically in a tortuous serpentine-like pattern comprising a plurality of U-formed portions arranged close to each other, see for example EP 1 177 802 and WO 02/15965. As the flow channel is established between at least two members, it is essential that these two members are held properly in place against each other, e.g. by bonding. However, in case of manufacturing imperfections or subsequent damage the close contact between the members may be incomplete such that fluid would flow in the gap between two adjacent members and consequently be able to short-cut portions of the tortuous path resulting in a (considerably) lower flow resistance.

Thus, in a first aspect the present invention provides a flow restrictor comprising a flow-restricting channel (in the following generally described as a "flow channel") formed between at least a first member and a second member arranged in contact with each other, the flow channel being arranged to form at least one generally U-formed portion with a pair of opposed first and second channel portions, and with a safety channel arranged between the opposed first and second channel portions, the safety channel providing a fluid communication with an exterior space relative to the flow restrictor. A flow restrictor of the invention may be formed integrally with the device in which it is implemented or it may be provided as a separate device or unit.

The safety channel establishes a flow path such that all or a portion of the fluid short-cutting the opposed channel portions in a given U-bend would be lead away from the flow channel, i.e. a "short-cut to the short-cut" is established. Preferably the safety channel is provided with a low flow resistance ensuring that substantially all fluid leaving the regular flow path is lead away, however, the exact flow resistance which will ensure this will be dependent upon both the characteristics and nature of the flow restrictor as well as the characteristics of other components of the drug delivery system. In this way drug infusion will stop and overdosing will be prevented. Indeed, in case underdosing is an issue, appropriate flow detection and alarm means may be provided.

In a preferred embodiment the flow channel as well as a plurality of safety channels is formed between two opposed surfaces. In such an arrangement a portion of a flow or safety channel is typically provided as an open trace or path formed in the surface of one member in combination with a planar portion of the other member which provides a "lid" thereby establishing a closed channel (indeed having one or two open ends). For a given flow restrictor the traces corresponding to different portions of the flow and safety channels may be provided in any of the two members in any desired configuration.

In a further preferred embodiment the flow channel as well as a plurality of safety channels is formed between an intermediate member sandwiched between two opposed surfaces. In such an arrangement through-going (i.e. perforating) traces are formed in the intermediate member, the through-going traces in combination with the opposed surface portions forming the flow channel and/or the safety channels. The intermediate member is preferably relative thin (e.g. a foil) in order to provide a narrow channel, correspondingly, the safety channels may be formed from traces in one or both of the opposed surfaces in combination with the foil. Although this arrangement results in the safety channels being arranged in a slightly different plane as the flow channel, for the purpose of the present disclosure they are considered to be arranged between the opposed portions of the individual U-portions.

As appears, numerous arrangements of the different channels or portions thereof can be contemplated just as channels formed from two, three or more members may be incorporated in a single flow restrictor.

When implementing the second principle, a fine capillary tube is used, the nature of which allows a relatively high flow resistance to be provided over a short length and at a low cost. As the capillary tube in most cases will be made from glass it is relatively fragile and it will be desirable to provide a supporting structure, however, in case a supporting structure (or any other structure) is provided surrounding the tube, a low resistance flow path may be established between the tube and the supporting structure, which in case of breakage of the fine tube may result in a dramatic reduction in flow resistance and a corresponding rise in the flow rate of the drive fluid.

Thus, a flow restrictor may comprise a capillary tube having an outer surface and first and second end portions, a supporting structure supporting at least the first and second end portions, where the supporting structure is arranged such that the outer surface along substantially the entire length of the capillary tube is in fluid communication with an exterior space relative to the flow restrictor. To provide the communication with an exterior space many different configurations of the supporting structure would be suitable. For example, the capillary tube may be arranged within a larger space of a supporting structure, e.g. within the cavity defined by an outer housing of a delivery device, the housing comprising an opening serving as a conduit to the exterior, or the supporting structure may provide a compartment, e.g. a channel, in which the capillary tube is arranged, the supporting structure further comprising conduit means (e.g. one or more channels) providing fluid communication between the compartment and the exterior.

In order to protect against breakage, the supporting structure may support the capillary tube at one or more points along the length thereof between the first and second end portions, however, it should still be assured that most of the outer surface is in fluid communication with the exterior space. The supporting structure may be provided with inlet and outlet openings in communication with the respective ends of the capillary tube.

The supporting structure may comprise a safety channel in flow communication with the space surrounding the capillary tube, which channel may be in direct flow communication with the exterior space. The supporting structure may be provided as an individual component or it may be provided as part of an overall structure, e.g. a housing component for a delivery device.

A flow restrictor may comprise a flow channel formed by a circumferential wall structure and having an inlet and an outlet in fluid communication with each other, wherein the circumferential wall structure along substantially the entire length of the flow channel has a portion in fluid communication with an exterior space relative to the flow restrictor. In exemplary embodiments the circumferential wall structure is formed by two or more members or by a capillary tube as described above.

For all of the above-described configurations, the exterior space relative to the flow restrictor may be represented by the "general space" surrounding an aggregate device in which the flow restrictor is incorporated or it may be an interior space in such a device. Indeed, in the latter case it should be prevented that any significant pressure builds up which would influence the function of the safety arrangement, e.g. by providing a relative large interior space or venting the interior space.

In a further aspect, the present invention provides a flow restrictor in which the risks associated with a flow restrictor is diminished by providing a composite unit comprising at least two independently manufactured flow restrictors arranged in series. By this arrangement the risk of substantial failure of the flow restructure is greatly diminished. For example, in case each of two flow restrictors provides 50% of the intended flow resistance, the (unlikely) total failure of one of the flow restrictors would still provide half the intended flow resistance and thereby "only" the double infusion rate.

Thus, in a further aspect the present invention provides a combined flow restrictor having a first flow restrictor of a first type, and a second flow restrictor of a second type arranged in series with the first flow restrictor. To exclude portions of a combined flow path which have only a neglectable flow resistance, e.g. the bores or conduits conducting fluid to and from the flow restrictor, it may be defined that each flow restrictor provides a "significant portion" of the combined flow resistance. For most practical purposes these structures are considered to posses a flow resistance of zero when determining the total flow resistance for a given flow path, e.g. between two cavities. More specifically, it may be defined that each of the first and second flow restrictors provides at least 5%, preferably at least 10%, and more preferably at least 25% of the combined flow resistance.

Each of the first and second flow restrictors may be selected from the group of flow restrictors comprising the types: capillary tube, micro opening, micro channel, micro bore, micro porous membrane and porous material.

Advantageously at least one further flow restrictor is arranged in series with the first and second flow restrictors, where each further flow restrictor may be of the first, second or a further type.

Preferably no single flow restrictor or type of flow restrictor provides more than approximately 50% of the combined flow resistance. For a combined flow restrictor comprising a plurality of flow restrictors or types of flow restrictors, preferably no single flow restrictor or type of flow restrictor provides more than approximately 25% of the combined flow resistance.

The individual flow restrictors may be arranged in individual members or one member may comprise two or more flow restrictors.

In the above described embodiments the flow restrictors have been characterized as being of different types, however, the same advantages can be achieved by providing at least two individual flow restrictors of the same type but manufactured independently of each other, e.g. using different manufacturing processes for manufacturing the same type of flow restrictor. The term "different manufacturing processes" includes manufacturing processes of the same type, e.g. a first flow restrictor hole may be manufactured by a first laser-drill set-up and a second flow restrictor hole may be manufactured by a second laser-drill set-up.

Thus, a method of manufacturing a combined flow restrictor comprises the steps of manufacturing a first flow restrictor in a first manufacturing process, manufacturing a second flow restrictor in a second manufacturing process independent of the first manufacturing process, and providing a combined flow restrictor device comprising the first and second flow restrictors arranged in series with each other. Corresponding to the discussion above, it may be defined that each flow restrictor provides at least 5%, preferably at least 10%, and more preferably at least 25% of the combined flow resistance.

Advantageously, the method comprises the further steps of manufacturing at least one further flow restrictor using the first, the second or a further manufacturing process, and providing a combined flow restrictor device comprising the first and second flow restrictors and at least one further flow restrictor, the flow restrictors being arranged in series with each other providing a combined flow restrictor in which the flow restrictor(s) from one manufacturing process preferably provide(s) at least 5%, more preferably at least 10% and most preferably at least 25% of the flow resistance for the combined flow resistor.

A further method of manufacturing a combined flow restrictor comprises the steps of manufacturing a plurality of flow restrictors using a plurality of independent manufacturing processes, and providing a combined flow restrictor device comprising the plurality of flow restrictors arranged in series with each other.

A further method of manufacturing a combined flow restrictor comprises the steps of manufacturing in a first manufacturing process a first member comprising a first flow restrictor or group of first flow restrictors, manufacturing in a second manufacturing process a second member comprising a second flow restrictor or group of second flow restrictors, and providing a combined flow restrictor device comprising the flow restrictors of the first and second member, at least one first and one second flow restrictor being arranged in series with each other providing a combined flow restrictor, the flow restrictor(s) from one manufacturing process preferably provide(s) at least 5%, more preferably at least 10%, and most preferably at least 25% of the flow resistance for a combined flow resistor.

In preferred embodiment the method comprises the steps of manufacturing a plurality of members each comprising at least one flow restrictor, thereby providing a plurality of flow restrictors, wherein at least two independent manufacturing processes are used for the manufacture of the flow restrictors, and providing a combined flow restrictor device comprising the plurality of flow restrictors.

For a flow restrictor manufactured in accordance with the above-described methods, preferably the flow restrictor(s) manufactured from a single manufacturing process provides approximately 50% or less of the combined flow resistance. For a combined flow restrictor comprising a plurality of flow restrictors or types of flow restrictors, preferably no single flow restrictor or type of flow restrictor provides more than approximately 25% of the combined flow resistance.

In exemplary embodiments a flow restrictor of the invention is incorporated into a delivery device comprising a housing, a first variable volume cavity containing a drive fluid, a flow restrictor comprising a flow channel, a second variable volume cavity in fluid communication with the first variable volume cavity through the flow channel, and a variable volume drug reservoir having in a situation of use an outlet means. The second variable volume cavity and the variable volume drug reservoir are arranged such that the volume of the drug reservoir diminishes when the volume of the second cavity increases. The delivery device further comprises drive means for expelling the drive fluid from the first to the second cavity through the flow restrictor, whereby drug is expelled from the drug reservoir through the outlet.

In further exemplary embodiments a flow restrictor is incorporated into a delivery device in which drug flow is controlled by directly expelling drug through a flow restrictor, the delivery device comprising a housing, a variable volume drug reservoir, a flow restrictor comprising a flow channel, and an outlet means in fluid communication with the first variable volume drug reservoir through the flow channel. A drive means is provided for expelling drug from the drug reservoir through the flow restrictor to the outlet means.

The outlet means may be adapted to be brought in fluid communication with infusion means (e.g. a catheter tubing or transcutaneous access means such as an infusion needle, a flexible infusion cannula or a plurality of micro-penetrators) or may comprise these. In the latter case the fluid communication may be established just prior to use, before or after the drug delivery device has been arranged on the user.

However, a delivery device of the above types may also be manufactured or offered to the user as a system in which individual components are combined with each other to provide an aggregate device. For example, it may be desirable to offer a system comprising a disposable, pre-filled drug unit, a durable drive-force providing unit and a disposable unit comprising the drive fluid and the flow restrictor. For such a system different flow restrictors providing different infusion rates in combination with a given drive-force could be offered.

Correspondingly, in an exemplary embodiment the present invention provides a fluid transmitting device comprising a first variable volume cavity containing a drive fluid, a flow restrictor as discussed and described above, a second variable volume cavity in fluid communication with the first variable volume cavity through the flow channel.

In a further exemplary embodiment the present invention provides a device comprising a prefilled variable volume drug reservoir having an outlet, and a flow restrictor as discussed and described above in fluid communication with the outlet of the reservoir.

In order to provide a fluid delivery device of the type using a flow restrictor in combination with a drive fluid which assures safe and easy identification whether infusion has started, a coloured drive fluid may be used. Thus, in a further aspect of the invention a delivery device is provided comprising a housing, a first variable volume cavity containing a drive fluid, a flow restrictor comprising a flow channel, a second variable volume cavity in fluid communication with the first variable volume cavity through the flow channel, and a variable volume drug reservoir having in a situation of use an outlet. The second variable volume cavity and the variable volume drug reservoir are arranged such that the volume of the drug reservoir diminishes when the volume of the second cavity increases. The device further comprises drive means for expelling the drive fluid from the first to the second cavity through the flow restrictor, whereby drug is expelled from the drug reservoir through the outlet, the housing comprising a transparent portion allowing the content of the second variable volume cavity or the flow restrictor to be viewed from outside the device, wherein the drive fluid is coloured (e.g. using a dye) for easy visual verification of its presence in the second variable volume cavity or the flow restrictor. As most liquid drugs are either transparent or milky (such as crystal-containing insulin) any "strong" colour such as red or blue may be used.

By this arrangement it is possible to identify the initial changes in the flow channel and/or the second cavity. Indeed, such a drive fluid arrangement may be used in combination with any type of flow restrictor. Advantageously, a flow restrictor of the above described type is used.

As used herein, the term "drug" is meant to encompass any drug-containing flowable medicine capable of being passed through a delivery means such as a hollow needle in a controlled manner, such as a liquid, solution, gel or fine suspension. Representative drugs include pharmaceuticals such as peptides, proteins, and hormones, biologically derived or active agents, hormonal and gene based agents, nutritional formulas and other substances in both solid (dispensed) or liquid form. In the description of the preferred embodiments reference will be made to the use of insulin. Correspondingly, the term "infusion" is meant to encompass any method of parenteral delivery to a subject.

### BRIEF DESCRIPTION OF THE DRAWINGS

In the following the invention will be further described with references to the drawings, wherein
fig. 1A is an exploded perspective view of a flow restrictor device,
fig. 1B shows in part a cross-section of a further flow restrictor device,
fig. 1C shows in a cross-sectional side view a further flow restrictor device,
fig. 1D shows a cross-sectional view along the line D-D in fig. 1C,
fig. 1E is an exploded perspective view of a further flow restrictor device,
fig. 1 F is an exploded perspective view of a further flow restrictor device based on the same principle as shown in fig. 1 E,
fig. 2A shows a perspective view of an infusion device in an initial state,
fig. 2B shows a perspective view of the infusion device of fig. 2A in an actuated state,
fig. 3 shows a "horizontal" cross-sectional view of the infusion device of figs. 2,
fig. 4 shows a first "vertical" cross-sectional view of the infusion device of figs. 2,
fig. 5 shows a second "vertical" cross-sectional view of the infusion device of figs. 2,
fig. 6 shows in detail a flow restrictor, and
fig. 7 shows in detail a subcutaneous infusion needle.

In the figures like structures are identified by like reference numerals.

### DESCRIPTION OF THE PREFERRED EMBODIMENTS

When in the following terms as "upper", "lower", "right" and "left" or similar relative expressions are used, these only refer to the appended figures and not to an actual situation of use. Further, the term "trace" is used to describe an "open" structure formed in a surface, e.g. a groove, whereas the term "channel" is used to describe a "closed" tubular structure which may have any cross-sectional configuration.

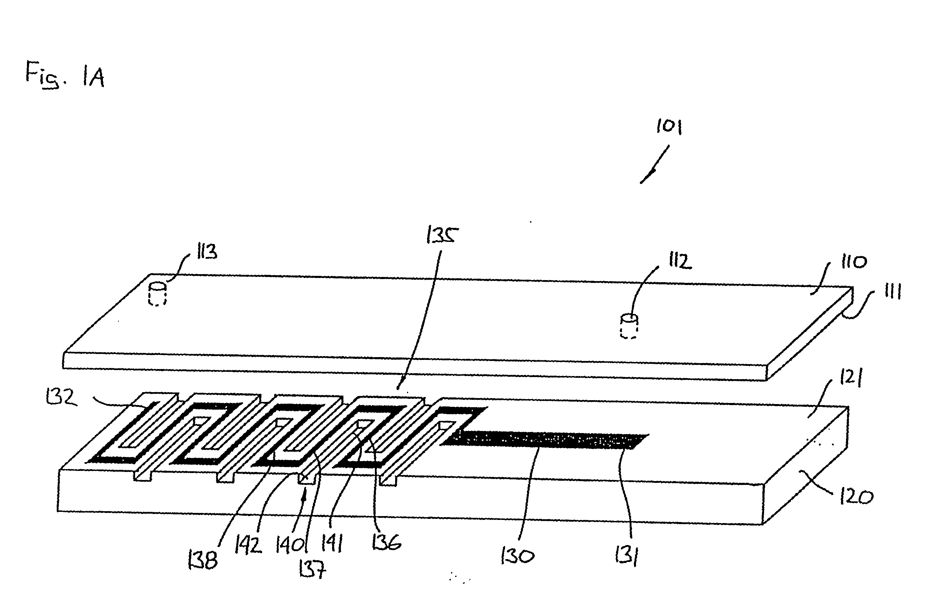

Fig. 1 A shows a schematic representation of a first embodiment of the invention. Correspondingly, the configuration of the different structures as well as there relative dimensions are intended to serve illustrative purposes only.

More specifically, a flow restrictor device 101 comprises an upper member 110 with a generally planar lower surface 111 (cannot be seen in fig. 1 A) and a lower member 120 with a generally planar upper surface 121. The upper member comprises first and second through-going bores 112, 113 serving as inlet respective outlet for the flow restrictor.

In the upper surface 121 is formed a flow trace 130 having an inlet end portion 131 and an outlet end portion 132 and a plurality of generally U-formed portions 135 each comprising a pair of opposed first and second "leg" portions 136, 137, the flow trace thereby forming a serpentine-like pattern. As appears, apart from the outermost legs, all the legs will form part of two neighbouring U-formed portions, i.e. legs 136, 137 will form a first U and legs 137, 138 will form a second U. The opposed portions may have any configuration (e.g. straight or curved) and may be arranged more or less in parallel with each other. For illustrative purposes is the trace 130 shown as a relatively broad structure, however, for most applications the trace will have a width and depth in the micrometer range.

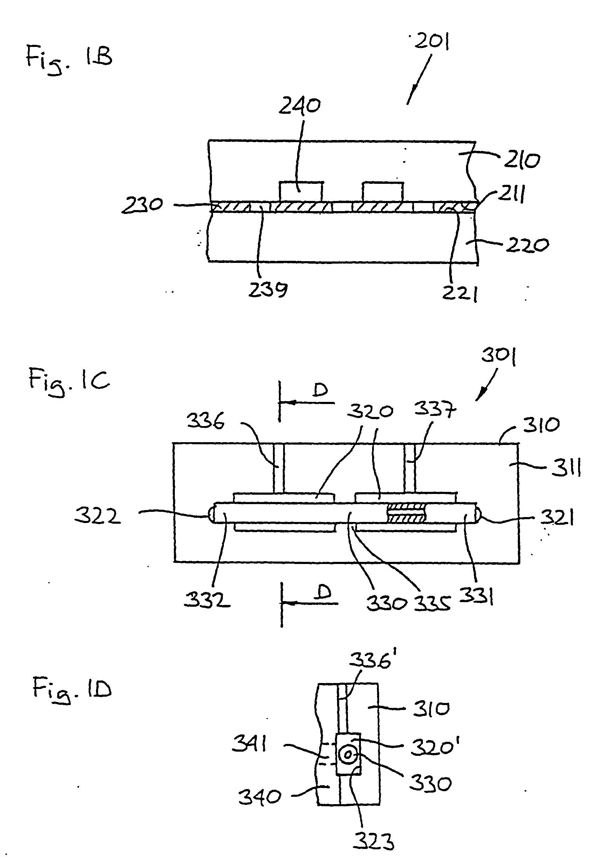

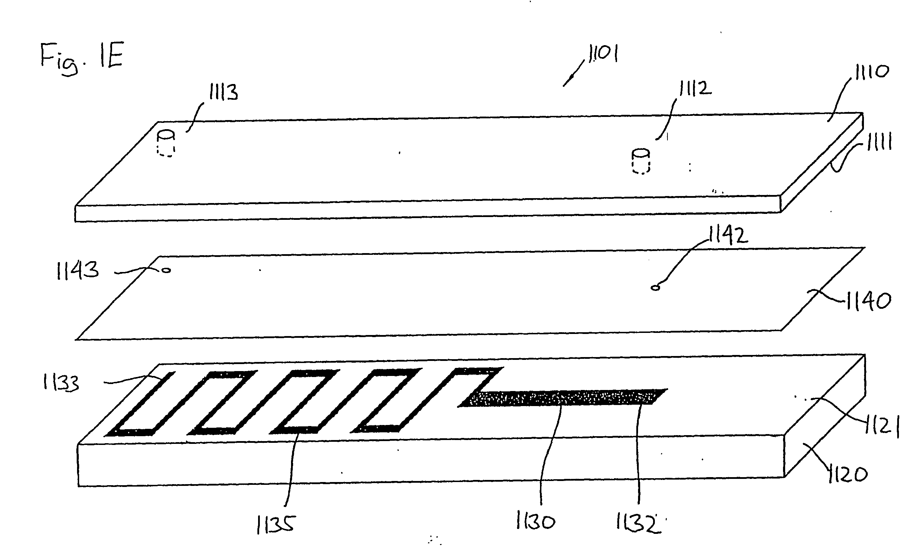

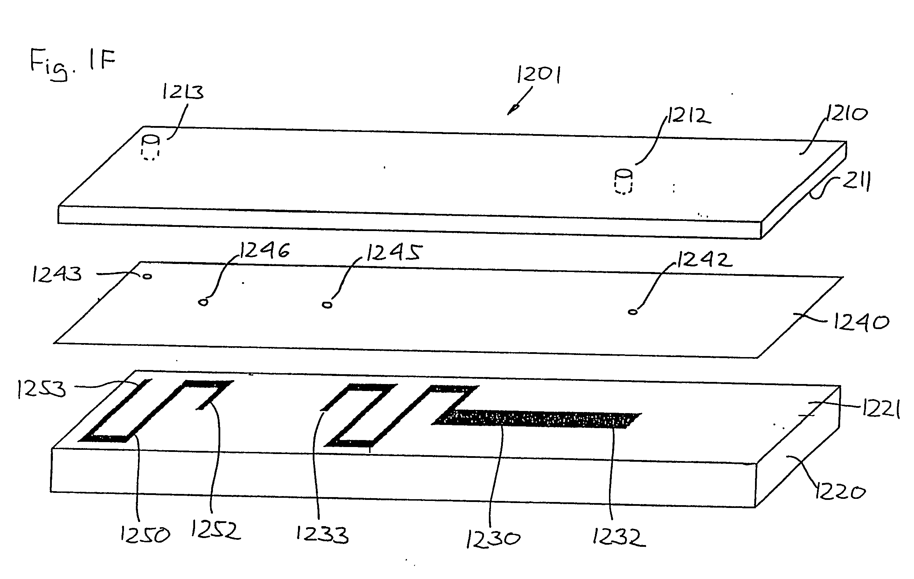

In the upper surface 121 between each of the opposed portions 136, 137 of the flow trace is formed safety traces 140 having a closed end 141 arranged in the vicinity of the closed end of the U and an opposed open end 142 in communication with the exterior. As schematically illustrated in fig. 1 is the cross-sectional area of the safety traces substantially larger than the flow trace.

In an assembled state (not shown) the two members 110, 120 are attached (e.g. bonded) to each other with the opposed surfaces in mating contact, whereby the flow trace and the safety traces will be "closed" to form a flow channel respectively a plurality of safety channels, the flow channel having an inlet end portion in fluid communication with the inlet opening 112 and an outlet end portion in fluid communication with the outlet opening 113. In this way the flow channel is formed by circumferential wall structures provided by the two members, wherein the circumferential wall structure along substantially the entire length of the flow channel has a portion (i.e. corresponding to the plane of the flow trace) in fluid communication with an exterior space relative to the flow restrictor.

As explained in detail above, should the bond between the two surfaces corresponding to a portion between two opposed legs 136, 137 of a U-formed portion 135 be defective, any fluid shortcutting the trace will be drained to the exterior through safety channel 140.

Fig. 1 B shows in part a schematic representation of a second embodiment of the invention. As in the first embodiment the flow restrictor device 201 comprises an upper member 210 with a lower surface 211 and a lower member 220 with an upper surface 221, however, in the second embodiment an intermediate foil member 230 is sandwiched between the two opposed surfaces, the three members being bonded to form a laminate structure. In the foil member is formed a through-going (i.e. perforating) trace 239 having essentially the same configuration as the trace 130 in the first embodiment. In the lower surface of the upper member is formed a plurality of safety traces 240 having essentially the same configuration as the safety traces 140 in the first embodiment. In this way a flow channel and its surrounding wall structure are formed from the three members in combination whereas the safety channels are formed between the first member and the intermediate member.

Figs. 1C and 1D show in schematic representation a third embodiment of a flow restrictor. The flow restrictor device 301 comprises a capillary tube 330 mounted in a first support member 310. The first support member 310 has a first surface 311 comprising a groove 320 adapted to accommodate the capillary tube. The groove is slightly longer than the tube providing an inlet 321 and an outlet 322, and is configured to securely engage and support the tube at its end portions 331, 332. In the shown embodiment optional supports 335 are provided to protect against breakage. To provide further support, the tube may be supported on the lower surface 323 of the groove corresponding to a narrow portion along its length (not shown).

The first support member further comprises two fluid conduits in the form of two safety traces 336, 337. In fig. 1D is the support member arranged against a second support member 340 whereby a support channel 320' is formed around the tube from which two safety channels 336' extend to the exterior. The second support member 340 comprises two bores 341 arranged in flow communication with the inlet respectively the outlet.

Fig. 1E shows a schematic representation of a further embodiment of a flow restrictor. Correspondingly, the configuration of the different structures as well as there relative dimensions are intended to serve illustrative purposes only.

More specifically, a flow restrictor device 1101 comprises an upper member 1110 with a generally planar lower surface 1111 (cannot be seen in fig. 1A), a lower member 1120 with a generally planar upper surface 1121, and a generally planar intermediate member 1140. The upper member comprises first and second through-going bores 1112, 1113 serving as inlet respective outlet for the flow restrictor.

In the upper surface is formed a flow trace 1130 having an inlet end portion 1132 and an outlet end portion 1133 and a plurality of generally U-formed portions 1135, the flow trace thereby forming a serpentine-like pattern. For illustrative purposes is the trace 1130 shown as a relatively broad structure, however, for most applications the trace will have a width and depth in the micrometer range. The trace may be formed using any suitable technology, e.g. injection moulding, stamping, etching or by means of a laser. Although the trace is shown as a unitary structure, it may comprise two or more portions manufactured using two or more different manufacturing processes, e.g. different lasers.

The intermediate member 1140 is in the form of a thin foil and comprises first and second openings 1142, 1143 serving as individual flow restrictors which are arranged to be in register with the bores 1112, 1113 in an assembled state of the device. The openings may be formed in the same or in two different processes. When a single manufacturing process is used, the two openings will belong to the same group of flow restrictors.

In an assembled state (not shown) the three members 1110, 1120, 1140 are attached (e.g. bonded) to each other in a sandwich construction with their opposed surfaces in mating contact, whereby the flow trace will be "closed" to form a flow channel, the flow channel having an inlet end portion 1132 serially in fluid communication with the first flow restrictor opening 1142 and the inlet opening 1112, and an outlet end portion 1133 serially in fluid communication with the second flow restrictor opening 1143 and the outlet opening 1113. As indicated in the figure, the inlet and outlet openings have a considerably larger diameter whereby their flow resistance are neglectable compared to the "real" flow restrictors 1142, 1130, 1143.

The flow resistance for the different flow restrictors may be chosen in accordance with the specific design of the combined flow restrictor device and the number of individual flow restrictors incorporated. For example, in case a first manufacturing process is used for the trace 1130 and a second manufacturing process is used for the two flow restrictor openings 1142, 1143, then a distribution of 25% for each of the openings and 50% for the trace may be suitable.

Fig. 1 F shows a schematic representation of a second embodiment of the invention. As in the fig. 1 E embodiment the flow restrictor device 1201 comprises an upper member 1210 with a lower surface 1211 and a lower member 1220 with an upper surface 1221 and an intermediate foil member 1240 sandwiched between the two opposed surfaces, the three members being adapted to be bonded together to form a laminate structure.

In the upper surface 1221 are formed first and second flow traces 1230, 1250 each having an inlet end portion 1232, 1252 and an outlet end portion 1233, 1253 and a plurality of generally U-formed portions 1235, 1255, the flow traces thereby forming serpentine-like patterns. In the lower surface 1211 are formed a third flow trace having inlet and outlet end portions (not to be seen), the trace being arranged corresponding to the area between the first and second flow traces. The three flow traces may be manufactured using one, two or three different manufacturing processes thereby defining three, two or one group(s) of flow restrictors.

The intermediate member 1240 member comprises first and second flow restrictors 1242, 1243 arranged to be in register with the bores 1112, 1113 in an assembled state of the device, as well as third and fourth flow restrictors 1245, 1246 arranged to be in register with end portions of the flow paths to thereby provide a serially connected flow path. The openings may be formed in the same or in different processes forming one or more group of flow restrictors.

In an assembled state (not shown) the three members 1210, 1220, 1240 are attached (e.g. bonded) to each other in a sandwich construction with their opposed surfaces in mating contact, whereby each flow trace will be closed to form a corresponding flow channel. When properly arranged in register with each other, a single combined flow restrictor is formed through the first opening 1242, the first flow channel 1230, the third opening 1245, the third flow channel, the fourth opening 1246, the second flow channel 1250 and the second opening 1243.

When a flow channel is established by a combination of a flow trace and an opposed "closing" surface, the flow channel (and thus the flow restrictor) is considered formed in the member in which the flow trace is formed.

Indeed, to the extent that a flow restrictor of the type having U-formed portions forming a serpentine-like pattern is used for a combined flow restrictor as discussed with reference to figs. 1 E and 1 F, the safety channels shown in fig. 1 A may advantageously be incorporated.

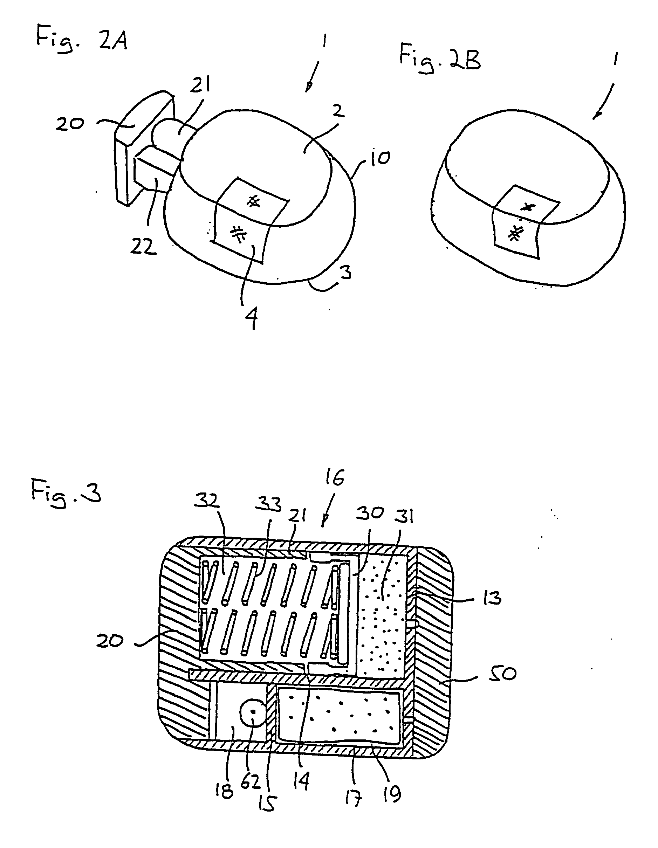

Fig. 2A shows a schematic representation of an embodiment of a delivery device. Correspondingly, the configuration of the different structures as well as their relative dimensions are intended to serve illustrative purposes only.

More specifically, fig. 2A shows an infusion device 1 comprising a housing 10 and there from protruding actuation button 20. The housing comprises an upper surface 2 and a lower surface 3 (not to be seen) adapted to be arranged against a skin surface of a user. The upper surface is provided with a transparent window 4 allowing the user to view a drug reservoir arranged within the housing. In fig. 28 the infusion device has been arranged against the skin of a user and the actuation button has pressed into the housing by the user thereby actuating the infusion device as will be explained in detail below.

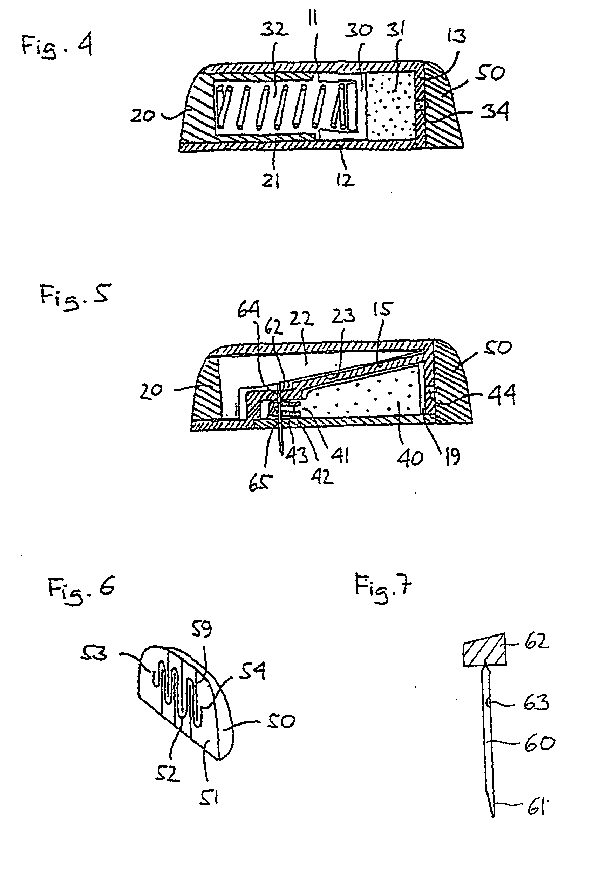

With reference to figs. 3-5 the general construction of the infusion device will be described. The housing comprises an upper wall 11, a lower planar base plate 12, side wall portions, an end wall 13 with an outer planar surface, and relative to the latter an opposed open end. Internally the housing comprises a first central wall 14 and a second oblique wall 15 in combination defining three compartments, a drive compartment 16, a reservoir compartment 17 and a needle compartment 18. The drive compartment forms a flat cylinder with an open proximal end and a substantially closed distal end. A piston 30 is slidingly arranged in the cylinder dividing the drive compartment in a distal fluid compartment 31 (corresponding to the above-described first cavity) filled with a viscous drive fluid (e.g. silicon oil), and a proximal spring compartment 32. The actuation button 20 comprises a skirt portion 21 slidingly received in the cylinder thereby closing the spring compartment. In the spring compartment are arranged two helical compression springs 33 acting on the piston, however, any compressible material or member providing a spring action or any other means providing or generating a force (e.g. gas generating means or a liquid/gas mixture) acting on the piston may be utilized. The actuation button further comprises a wedge portion 22 to be received in the needle compartment.

As best seen in fig. 5 the reservoir compartment comprises a flexible drug reservoir 40 with an insulin-containing drug formulation. The reservoir is preferably manufactured from a transparent material allowing the user to view and control the drug through the window 4. In the initial state, i.e. before any drug has been expelled from the infusion device, the reservoir has a configuration substantially corresponding to the configuration of the reservoir compartment, thereby forming a neglectable cavity 19 (or dead-space) between the two components. In case an air filled dead space is not acceptable, the space may be filled with a fluid (for illustrative purposes is the gap between the reservoir and the reservoir compartment relatively large). As appears, the dead-space represents the above-described second cavity in a substantially fully collapsed state. Inside the drug reservoir is arranged a U-formed membrane element 41 formed from a self-sealing material and comprising upper and lower membrane portions 42, 43. In the end portion 13 is formed an outlet opening 34 from the fluid compartment and an inlet opening 44 to the reservoir compartment.

The infusion device further comprises a flow restrictor member 50 (see fig. 6) comprising a planer surface 51 in which a serpentine trace 52 is formed between proximal and distal end portions 53, 54. Between the opposed legs of the individual serpentine U-formed portions are arranged safety traces 59. The flow restrictor member 50 is bonded to the outer planar surface of the housing end portion with the proximal and distal end portions in register with the outlet 34 respectively the inlet openings 44. In this way a flow restrictor channel with safety channels is formed between the two openings. Alternatively, any of the other flow restrictors disclosed above may be utilized. As appears, the resistance of the flow restrictor, the viscosity of the drive fluid and the force provided by the compressed springs will determine the rate at which the drive fluid will be forced through the flow restrictor to the reservoir compartment.

The infusion device further comprises a hollow subcutaneous infusion needle 60 as shown in fig. 7, comprising a distal pointed end 61 adapted to be introduced through a skin surface, a closed proximal end at which a needle wedge 62 is formed. In the body of the needle an opening 63 is formed in flow communication with interior of the needle. The proximal end of the needle is arranged in the needle compartment and with the needle body protruding through an opening 64 formed in the first wall 15 into the reservoir compartment and further into the reservoir. In the initial state (as supplied to the user and not shown in fig. 5) the needle penetrates the upper membrane portion 42 with the distal end 61 arranged between the upper and lower membrane portions 42, 43 inside the reservoir.

Next, with reference to figs. 4 and 5 actuation of the infusion device will be described. When the device has been positioned on a skin surface (preferably the lower surface comprises an adhesive coating) the user actuates the device by fully depressing the actuation button 20 until it locks in place in a recessed position (locking means arranged between the button and the housing is not shown in the figs.) whereby simultaneously the springs 33 are compressed and the wedge portion 22 is moved into the needle compartment. The wedge portion comprises a lower oblique surface 23 in sliding contact with the needle wedge 62 whereby the wedge portion forces the needle downwardly as it is pressed into housing. By this action the pointed distal needle end 61 penetrates the lower membrane portion 43 and is forced out through an opening 65 formed in the base portion. As the infusion device is attached to the skin surface of the user, the infusion needle is hereby introduced through the skin. When the needle is in its fully extended position, the needle opening 63 is positioned between the two membrane portions whereby a fluid communication is established from the drug reservoir to the user. At the same time the drive fluid starts to be expelled from the fluid compartment 31 and through the flow restrictor to the cavity portion 19 of the reservoir compartment 17 where it gradually will compress the flexible reservoir and thereby force out the therein contained insulin-containing drug through the needle and into the user.

Initially air will be expelled from the needle just as air trapped in the flow restrictor and around the drug reservoir (if any) may result in an initial higher infusion rate, however, these effects will be neglectable.

In the shown embodiment the expelling means in form of springs 33 are "energized" during actuation of the device, however, to reduce the force needed to actuate the button 20 the spring means 33 may be pre-tensioned and the drive fluid 31 correspondingly pre-pressurized, whereby alone puncturing of the reservoir by the needle will actuate the expelling means and thereby start infusion.

In order to provide a fluid delivery device of the type using a flow restrictor in combination with a drive fluid which assures safe and easy identification whether infusion has started, a coloured drive fluid may be used. More specifically, the housing may comprise a transparent portion allowing the content of the second variable volume cavity or the flow restrictor to be viewed from outside the delivery device, and the drive fluid may be coloured (e.g. using a dye) for easy visual verification of its presence in the second variable volume cavity or the flow restrictor. By this arrangement it is possible to identify the initial changes in the flow channel and/or the second cavity. Indeed, such a drive fluid arrangement may be used in combination with any type of flow restrictor.

In the above description of the preferred embodiments, the different structures providing the desired relations between the different components just as the means providing the described functionality for the different components (i.e. force generating means, flow restrictor, flexible reservoir etc.) have been described to a degree to which the concept of the present invention will be apparent to the skilled reader. The detailed construction and specification for the different structures are considered the object of a normal design procedure performed by the skilled person along the lines set out in the present specification.

## Claims

1. A flow restrictor (101) comprising:
- a flow channel (130) formed between at least a first member (110) and a second member (120) arranged in engagement with each other,
- the flow channel having an inlet end portion (131) in fluid communication with an inlet (112) opening and an outlet end portion (132) in fluid communication with an outlet opening (113),
- the flow channel comprising a generally U-formed portion (135) with a pair of opposed first and second channel portions (136, 137), **characterized by**
- a safety channel (140) arranged between the opposed first and second channel portions,
- the safety channel comprising an end portion (142) in fluid communication with an exterior space relative to the flow restrictor.

2. A flow restrictor as defined in claim 1, comprising a plurality of generally U-formed portions, each with a pair of opposed first and second channel portions.

3. A flow restrictor as defined in claim 2, comprising a plurality of safety channels arranged between at least a portion of the opposed first and second channel portions.

4. A flow restrictor as defined in any of claims 1-3, wherein:
- the first member comprises a first surface portion (111) and the second member comprises a second surface portion (121), the first and second surface portions being arranged in opposed engagement with each other, and wherein
- traces (130, 140) are formed in at least one of the first and second surface portions, the traces in combination with an opposed surface portion forming the flow channel and the safety channel(s).

5. A flow restrictor as defined in claim 4, wherein the surface traces are formed in one of the first and second surface portions.

6. A flow restrictor as defined in any of claims 1-3, wherein:
- the first member (210) comprises a first surface portion (211) and the second member (220) comprises a second surface portion (221), the flow restrictor further comprising an intermediate member (230) arranged between the first and second surface portions and in engagement therewith, and wherein
- at least one through-going trace is (239) formed in the intermediate member, the through-going trace(s) in combination with opposed surface portions forming at least a portion of the flow channel and/or the safety channel(s).

7. A flow restrictor as defined in claim 6, wherein traces (240) are formed in at least one of the first and second surface portions (211, 221), the traces in combination with opposed portions of the intermediate member forming at least a portion of the flow channel and/or the safety channel(s).

8. A flow restrictor as defined in any of the previous claims, wherein the inlet and outlet openings (112, 113) are formed in the first and/or second member.

9. A delivery device (1) comprising:
- a first variable volume cavity (31) containing a drive fluid,
- a flow restrictor (50, 52) as defined in any of the previous claims,
- a second variable volume cavity (19) in fluid communication with the first variable volume cavity through the flow channel,
- a variable volume drug reservoir (40) having in a situation of use an outlet means,
- the second variable volume cavity and the variable volume drug reservoir being arranged such that the volume of the drug reservoir diminishes when the volume of the second cavity increases, and
- drive means (33) for expelling the drive fluid from the first to the second cavity through the flow restrictor, whereby drug is expelled from the drug reservoir through the outlet.

10. A delivery device as defined in claim 9, wherein the housing comprises a transparent portion allowing the content of the second variable volume cavity or the flow restrictor to be viewed from outside the device, wherein the drive fluid is coloured for easy visual verification of its presence in the second variable volume cavity or the flow restrictor.

11. A delivery device comprising:
- a housing,
- a variable volume drug reservoir,
- a flow restrictor as defined in any of the previous claims,
- an outlet means in fluid communication with the first variable volume drug reservoir through the flow channel,
- drive means for expelling drug from the drug reservoir through the flow restrictor to the outlet means.

12. A fluid transmitting device comprising:
- a first variable volume cavity containing a drive fluid,
- a flow restrictor as defined in any of the previous claims,
- a second variable volume cavity in fluid communication with the first variable volume cavity through the flow channel.

13. A drug device comprising:
- a pre-filled variable volume drug reservoir having an outlet,
- a flow restrictor as defined in any of the previous claims in fluid communication with the outlet of the reservoir.

14. A combined flow restrictor comprising:
- a first flow restrictor (1130) of a first type as defined in claim 1,
- a second flow restrictor (1142) of a second type arranged in series with the first flow restrictor.

15. A combined flow restrictor as defined in claim 14, wherein each of the first and second flow restrictors is selected from the group of flow restrictors comprising the types capillary tube, micro opening and micro channel.

16. A combined flow restrictor as defined in claim 14 or 15, further comprising:
- at least one further flow restrictor (1143) arranged in series with the first and second flow restrictors,
- each further flow restrictor being of the first, second or a further type.

## Patentansprüche

1. Flussbeschränkungsvorrichtung (101), umfassend:
einen Fließkanal (130), der zwischen mindestens einem ersten Element (110) und einem zweiten Element (120), die miteinander in Eingriff angeordnet sind, ausgebildet ist,
- wobei der Fließkanal einen Einlassendteil (131) in Fluidkommunikation mit einer Einlassöffnung (112) und einen Auslassendteil (132) in Fluidkommunikation mit einer Auslassöffnung (113) aufweist,
- wobei der Fließkanal einen im Allgemeinen U-förmigen Teil (135) mit einem Paar einander gegenüberliegenden ersten und zweiten Kanalteilen (136, 137) umfasst, **gekennzeichnet durch**
- einen Sicherheitskanal (140), der zwischen den einander gegenüberliegenden ersten und zweiten Kanalteilen angeordnet ist,
- wobei der Sicherheitskanal einen Endteil (142) in Fluidkommunikation mit einem Außenraum in Bezug auf die Flussbeschränkungsvorrichtung umfasst.

2. Flussbeschränkungsvorrichtung wie in Anspruch 1 definiert, umfassend eine Mehrzahl im Allgemeinen U-förmiger Teile, jedes mit einem Paar einander gegenüberliegender erster und zweiter Kanalteile.

3. Flussbeschränkungsvorrichtung wie in Anspruch 2 definiert, umfassend eine Mehrzahl an Sicherheitskanälen, die zwischen mindestens einem Teil der einander gegenüberliegenden ersten und zweiten Kanalteile angeordnet sind.

4. Flussbeschränkungsvorrichtung wie in einem der Ansprüche 1-3 definiert:
- wobei das erste Element einen ersten Oberflächenteil (111) umfasst und das zweite Element einen zweiten Oberflächenteil (121) umfasst, wobei der erste und der zweite Oberflächenteil in einander gegenüberliegendem Eingriff miteinander angeordnet sind und
- wobei Spurlinien (130, 140) in mindestens einem der ersten und zweiten Oberflächenteile ausgebildet sind, wobei die Spuren mit einem gegenüberliegenden Oberflächenteil den Fließkanal und den (die) Sicherheitskanal (Sicherheitskanäle) ausbilden.

5. Flussbeschränkungsvorrichtung wie in Anspruch 4 definiert, wobei die Oberflächenspurlinien in einem der ersten und zweiten Oberflächenteile ausgebildet sind.

6. Flussbeschränkungsvorrichtung wie in einem der Ansprüche 1-3 definiert:
- wobei das erste Element (210) einen ersten Oberflächenteil (211) umfasst und das zweite Element (220) einen zweiten Oberflächenteil (221) umfasst, wobei die Flussbeschränkungsvorrichtung des Weiteren ein Zwischenelement (230) umfasst, das zwischen den ersten und zweiten Oberflächenteilen und in Eingriff damit angeordnet ist, und
- wobei mindestens eine durchlaufende Spurlinie (239) im Zwischenelement ausgebildet ist, wobei die durchlaufende(n) Spurlinie(n) in Kombination mit den gegenüberliegenden Oberflächenteilen mindestens einen Teil des Fließkanals und/oder des (der) Sicherheitskanals (Sicherheitskanäle) ausbildet (ausbilden).

7. Flussbeschränkungsvorrichtung wie in Anspruch 6 definiert, wobei Spurlinien (240) in mindestens einem der ersten und zweiten Oberflächenteile (211, 221) ausgebildet sind, wobei die Spurlinien in Kombination mit einander gegenüberliegenden Teilen des Zwischenelements mindestens einen Teil des Fließkanals und/oder des (der) Sicherheitskanals (Sicherheitskanäle) ausbilden.

8. Flussbeschränkungsvorrichtung wie in einem der vorangehenden Ansprüche definiert, wobei die Einlass- und Auslassöffnungen (112, 113) im ersten und/oder zweiten Element ausgebildet sind.

9. Abgabevorrichtung (1), umfassend:
- einen ersten Hohlraum (31) mit variierbarem Volumen, der ein Antriebsfluid enthält,
- eine wie in einem der vorangehenden Ansprüche definierte Flussbeschränkungsvorrichtung (50, 52)
- einen zweiten Hohlraum (19) mit variierbarem Volumen in Fluidkommunikation mit dem ersten Hohlraum mit variierbarem Volumen durch den Fließkanal,
- einen Arzneimittelbehälter (40) mit variierbarem Volumen, der in einer Verwendungssituation ein Auslassmittel aufweist,
- wobei der zweite Hohlraum mit variierbarem Volumen und der Arzneimittelbehälter mit variierbarem Volumen derart angeordnet sind, dass das Volumen des Arzneimittelbehälters abnimmt, wenn das Volumen des zweiten Hohlraums zunimmt, und
- ein Antriebsmittel (33) zum Ausstoßen des Antriebsfluids aus dem ersten zu dem zweiten Hohlraum durch die Flussbeschränkungsvorrichtung, wodurch Arzneimittel aus dem Arzneimittelbehälter durch den Auslass ausgestoßen wird.

10. Abgabevorrichtung wie in Anspruch 9 definiert, wobei das Gehäuse einen transparenten Teil umfasst, der es ermöglicht, dass der Inhalt des zweiten Hohlraums mit variierbarem Volumen oder der Flussbeschränkungsvorrichtung von der Außenseite der Vorrichtung her sichtbar ist, wobei das Antriebsfluid zum leichten visuellen Erkennen seiner Gegenwart im zweiten Hohlraum mit variierbarem Volumen oder in der Flussbeschränkungsvorrichtung gefärbt ist.

11. Abgabevorrichtung, umfassend:
- ein Gehäuse,
- einen Arzneimittelbehälter mit variierbarem Volumen,
- eine wie in einem der vorangehenden Ansprüche definierte Flussbeschränkungsvorrichtung,
- ein Auslassmittel in Fluidkommunikation mit dem ersten Arzneimittelbehälter mit variierbarem Volumen durch den Fließkanal,
- ein Antriebsmittel zum Ausstoßen des Arzneimittels aus dem Arzneimittelbehälter durch die Flussbeschränkungsvorrichtung zum Auslassmittel.

12. Fluidübertragungsvorrichtung, umfassend:
- einen ersten Hohlraum mit variierbarem Volumen, der ein Antriebsfluid enthält,
- eine wie in einem der vorangehenden Ansprüchen definierte Flussbeschränkungsvorrichtung
- einen zweiten Hohlraum mit variierbarem Volumen in Fluidkommunikation mit dem ersten Hohlraum mit variierbarem Volumen durch den Fließkanal.

13. Arzneimittelvorrichtung, umfassend:
- einen vorgefüllten Arzneimittelbehälter mit variierbarem Volumen mit einem Auslass,
- eine wie in einem der vorangehenden Ansprüche definierte Flussbeschränkungsvorrichtung in Fluidkommunikation mit dem Auslass des Behälters.

14. Kombinierte Flussbeschränkungsvorrichtung, umfassend:
- eine erste Flussbeschränkungsvorrichtung (1130) eines wie in Anspruch 1 definierten ersten Typs,
- eine zweite Flussbeschränkungsvorrichtung (1142) eines zweiten Typs, die in Reihe mit der ersten Flussbeschränkungsvorrichtung angeordnet ist.

15. Kombinierte Flussbeschränkungsvorrichtung wie in Anspruch 14 definiert, wobei jede der ersten und der zweiten Flussbeschränkungsvorrichtung ausgewählt ist aus der Gruppe von Flussbeschränkungsvorrichtungen, die die Typen Kapillarrohr, Mikroöffnung und Mikrokanal umfassen.

16. Kombinierte Flussbeschränkungsvorrichtung wie in Anspruch 14 oder 15 definiert, des Weiteren umfassend:
- mindestens eine weitere Flussbeschränkungsvorrichtung (1143), die in Reihe mit der ersten und der zweiten Flussbeschränkungsvorrichtung angeordnet ist,
- wobei jede weitere Flussbeschränkungsvorrichtung vom ersten, zweiten oder von einem weiteren Typ ist.

## Revendications

1. Réducteur de débit (101) comprenant :
- un canal d'écoulement (130) formé entre au moins un premier élément (110) et un second élément (120) disposés en engagement l'un avec l'autre,
- le canal d'écoulement comportant une partie d'extrémité d'entrée (131) en communication fluidique avec une ouverture d'entrée (112) et une partie d'extrémité de sortie (132) en communication fluidique avec une ouverture de sortie (113),
- le canal d'écoulement comprenant une partie globalement en forme de U (135) dotée d'une paire de première et seconde parties de canal opposées (136, 137), **caractérisé par**
- un canal de sécurité (140) disposé entre les première et seconde parties de canal opposées,
- le canal de sécurité comprenant une partie d'extrémité (142) en communication fluidique avec un espace extérieur par rapport au réducteur de débit.

2. Réducteur de débit selon la revendication 1, comprenant une pluralité de parties globalement en forme de U, chacune étant dotée d'une paire de première et seconde parties de canal opposées.

3. Réducteur de débit selon la revendication 2, comprenant une pluralité de canaux de sécurité disposés entre au moins une partie des première et seconde parties de canal opposées.

4. Réducteur de débit selon l'une quelconque des revendications 1 à 3, dans lequel :
- le premier élément comprend une première partie formant surface (111) et le second élément comprend une seconde partie formant surface (121), les première et seconde parties formant surfaces étant disposées en engagement opposé l'une avec l'autre, et dans lequel
- des traces (130, 140) sont formées dans au moins l'une des première et seconde parties formant surfaces, les traces formant le canal d'écoulement et le canal ou les canaux de sécurité en combinaison à une partie formant surface opposée.

5. Réducteur de débit selon la revendication 4, dans lequel les traces de surface sont formées dans l'une des première et seconde parties formant surfaces.

6. Réducteur de débit selon l'une quelconque des revendications 1 à 3, dans lequel :
- le premier élément (210) comprend une première partie formant surface (211) et le second élément (220) comprend une seconde partie formant surface (221), le réducteur de débit comprenant en outre un élément intermédiaire (230) disposé entre les première et seconde parties formant surfaces et en engagement avec celles-ci, et dans lequel
- au moins une trace traversante (239) est formée dans l'élément intermédiaire, la trace ou les traces traversante(s) formant au moins une partie du canal d'écoulement et/ou du canal ou des canaux de sécurité en combinaison aux parties formant surfaces opposées.

7. Réducteur de débit selon la revendication 6, dans lequel des traces (240) sont formées dans au moins l'une des première et seconde parties formant surfaces (211, 221), les traces formant au moins une partie du canal d'écoulement et/ou du canal ou des canaux de sécurité en combinaison aux parties opposées de l'élément intermédiaire.

8. Réducteur de débit selon l'une quelconque des revendications précédentes, dans lequel les ouvertures d'entrée et de sortie (112, 113) sont formées dans le premier et/ou le second élément.

9. Dispositif de distribution (1) comprenant :
- une première cavité à volume variable (31) contenant un fluide d'entraînement,
- un réducteur de débit (50, 52) selon l'une quelconque des revendications précédentes,
- une seconde cavité à volume variable (19) en communication fluidique avec la première cavité à volume variable par l'intermédiaire du canal d'écoulement,
- un réservoir de médicament à volume variable (40) comportant un moyen de sortie dans une situation d'utilisation,
- la seconde cavité à volume variable et le réservoir de médicament à volume variable étant disposés de sorte que le volume du réservoir de médicament diminue lorsque le volume de la seconde cavité augmente, et
- un moyen d'entraînement (33) pour expulser le fluide d'entraînement de la première à la seconde cavité par l'intermédiaire du réducteur de débit, moyennant quoi le médicament est expulsé du réservoir de médicament à travers la sortie.

10. Dispositif de distribution selon la revendication 9, dans lequel le boîtier comprend une partie transparente permettant au contenu de la seconde cavité à volume variable ou du réducteur de débit d'être vu depuis l'extérieur du dispositif, dans lequel le fluide d'entraînement est coloré pour une vérification visuelle facile de sa présence dans la seconde cavité à volume variable ou le réducteur de débit.

11. Dispositif de distribution comprenant :
- un boîtier,
- un réservoir de médicament à volume variable,
- un réducteur de débit selon l'une quelconque des revendications précédentes,
- un moyen de sortie en communication fluidique avec le premier réservoir de médicament à volume variable par l'intermédiaire du canal d'écoulement,
- un moyen d'entraînement pour expulser le médicament du réservoir de médicament par l'intermédiaire du réducteur de débit vers le moyen de sortie.

12. Dispositif de transmission de fluide comprenant :
- une première cavité à volume variable contenant un fluide d'entraînement,
- un réducteur de débit selon l'une quelconque des revendications précédentes,
- une seconde cavité à volume variable en communication fluidique avec la première cavité à volume variable par l'intermédiaire du canal d'écoulement.

13. Dispositif à médicament comprenant :
- un réservoir de médicament à volume variable pré-rempli comportant une sortie,
- un réducteur de débit selon l'une quelconque des revendications précédentes en communication fluidique avec la sortie du réservoir.

14. Réducteur de débit combiné comprenant :
- un premier réducteur de débit (1130) d'un premier type selon la revendication 1,
- un second réducteur de débit (1142) d'un second type disposé en série avec le premier réducteur de débit.

15. Réducteur de débit combiné selon la revendication 14, dans lequel chacun des premier et second réducteurs de débit est sélectionné dans le groupe de réducteurs de débit comprenant les types à tube capillaire, à micro-ouverture et à micro-canal.

16. Réducteur de débit combiné selon la revendication 14 ou 15, comprenant en outre :
- au moins un autre réducteur de débit (1143) disposé en série avec les premier et second réducteurs de débit,
- chaque autre réducteur de débit étant du premier, deuxième ou d'un autre type.
